# EUROPÄISCHE PATENTSCHRIFT

(11) **EP 2 956 151 B1**
(45) Veröffentlichungstag und Bekanntmachung des Hinweises auf die Patenterteilung: **11.01.2017**
(21) Anmeldenummer: 14706504.9
(22) Anmeldetag: 11.02.2014
(51) Int. Cl.: A61K 36/28, A61P 25/28

(54) **VERWENDUNG VON EXTRAKTEN AUS CALENDULA ZUR BEHANDLUNG UND VORBEUGUNG VON STÖRUNGEN UND BEEINTRÄCHTIGUNGEN KOGNITIVER UND MENTALER FUNKTIONEN**
USE OF EXTRACTS FROM CALENDULA FOR THE TREATMENT AND PREVENTION OF DISORDERS AND IMPAIRMENTS OF COGNITIVE AND MENTAL FUNCTIONS
UTILISATION D'EXTRAITS DE CALENDULA POUR LE TRAITEMENT ET LA PRÉVENTION DE TROUBLES ET DE COMPROMISSIONS DE FONCTIONS COGNITIVES ET MENTALES

(30) Priorität: 14.02.2013 DE 102013202368
(43) Veröffentlichungstag der Anmeldung: 23.12.2015
(73) Patentinhaber: Dr. Willmar Schwabe GmbH & Co. KG, 76227 Karlsruhe (DE)
(72) Erfinder: ERDELMEIER, Clemens, 76139 Karlsruhe (DE); HAUER, Hermann, 76228 Karlsruhe (DE); KOCH, Egon, 76229 Karlsruhe (DE); NÖLDNER, Michael, 76139 Karlsruhe (DE)
(74) Vertreter: Adam, Holger
(86) Internationale Anmeldenummer: PCT/EP2014/052617
(87) Internationale Veröffentlichungsnummer: WO 2014/124928

(56) Entgegenhaltungen:
- WO-A1-2005/117924
- WO-A1-2011/098448
- US-A1- 2008 063 658
- KURKIN V A ET AL: "Flavonoids from Calendula officinalis flowers", MEDICINAL & AROMATIC PLANTS ABSTRACTS, SCIENTIFIC PUBLISHERS, SCIENTIFIC PUBLISHERS, NEW DELHI - INDIA, Bd. 29, Nr. 6, 1. Dezember 2007 (2007-12-01), XP018023641, ISSN: 0250-4367
- DATABASE WPI Week 200130 Thomson Scientific, London, GB; AN 2001-282553 XP002723030, & CN 1 280 842 A (LI Y) 24. Januar 2001 (2001-01-24)
- HAN C K ET AL: "Cognition-enhancing and neuroprotective effects of hederacolchiside-E from Pulsatilla koreana", MEDICINAL & AROMATIC PLANTS ABSTRACTS, SCIENTIFIC PUBLISHERS, SCIENTIFIC PUBLISHERS, NEW DELHI - INDIA, Bd. 29, Nr. 6, 1. Dezember 2007 (2007-12-01), XP018022578, ISSN: 0250-4367
- HILLHOUSE B J ET AL: "Acetylcholine esterase inhibitors in Rhodiola rosea", MEDICINAL & AROMATIC PLANTS ABSTRACTS, SCIENTIFIC PUBLISHERS, SCIENTIFIC PUBLISHERS, NEW DELHI - INDIA, Bd. 27, Nr. 2, 1. März 2005 (2005-03-01), XP018004364, ISSN: 0250-4367

## Beschreibung

Die vorliegende Erfindung betrifft die Verwendung von Extrakten aus *Calendula* zur Behandlung und Vorbeugung bzw. Therapie und Prophylaxe von Störungen und Beeinträchtigungen kognitiver und mentaler Funktionen und zur Verbesserung mentaler und kognitiver Funktionen, insbesondere zur Behandlung und Vorbeugung demenzieller Syndrome sowie deren Frühformen und Vorstufen.

Weiterhin betrifft die vorliegende Erfindung Extrakte aus Calendula zur Verwendung in der Behandlung und Vorbeugung von Störungen und Beeinträchtigungen kognitiver und mentaler Funktionen, insbesondere zur Behandlung und Vorbeugung demenzieller Syndrome sowie deren Frühformen und Vorstufen.

Unter kognitiven und mentalen Funktionen versteht man grundsätzlich alle geistigen Fähigkeiten und Leistungen. Dazu zählen u.a. Gedächtnis- und Aufmerksamkeitsfunktionen, verbale Leistungen (Sprache) und visuelle Kognitionen (etwa räumliches Denken). Diese Leistungen sind in der modernen Neuropsychologie gut quantifizierbar und interindividuell vergleichbar. Störungen der mentalen Leistungsfähigkeit führen zu einer, je nach Grad der Veränderung, ansteigenden Belastung der betroffenen Person, aber auch der Bezugspersonen. Erste Beeinträchtigungen sind häufig eine verstärkte Vergesslichkeit, die sich dann im Laufe der Zeit steigern kann bis zur völligen Demenz und einer damit einhergehenden Pflegebedürftigkeit.

Die Behandlung von Demenzerkrankungen, vor allem vom Alzheimertyp erfolgt zur Zeit bevorzugt mit Extrakten aus *Ginkgo biloba* Blättern oder mit Arzneimitteln, die die Neurotransmission cholinerger Neurone steigern, z.B. Donepezil, oder die die Neurotransmission exzitatorischer Neurone hemmen, z.B. Memantine, wobei die therapeutischen Möglichkeiten von Donepezil und Memantine durch deren Nebenwirkungsspektrum limitiert sind.

Auch die Verwendung von Isorhamnetintriglycosiden und diese enthaltende Pflanzenextrakte, beispielsweise von Opuntia-Arten, zur Behandlung und Prophylaxe von neurologischen Erkrankungen, die mit einer Störung mentaler Funktionen verbunden sind, ist aus WO2011/098448 bekannt.

Aufgabe der vorliegenden Erfindung ist es nun ein Mittel bereitzustellen, das bei innerer Anwendung Störungen und Beeinträchtigungen kognitiver und mentaler Funktionen behandelt oder diesen vorbeugt und gleichzeitig gut verträglich ist.

Diese Aufgabe wird erfindungsgemäß gelöst durch die Verwendung von Extrakten aus Calendula zur Behandlung und Vorbeugung von Störungen und Beeinträchtigungen kognitiver und mentaler Funktionen, insbesondere von demenziellen Syndromen, insbesondere von leichten bis schweren Formen der Alzheimer-Demenz, der vaskulären Demenz und deren Mischformen, sowie von Frühformen und Vorstufen demenzieller Syndrome, insbesondere von leichten kognitiven Beeinträchtigungen (MCI, "mild cognitive impairment"), kognitiven Beeinträchtigungen, die noch keine Demenz darstellen (CIND, "cognitive impairment no dementia") sowie objektiv oder subjektiv empfundenen Gedächtnisbeeinträchtigungen und Beeinträchtigungen anderer kognitiver Leistungskomponenten, Apathie, Antriebsmangel und anderen demenziellen Symptomen. Bei den anderen subjektiv und/oder objektiv beeinträchtigten kognitiven Leistungskomponenten handelt es sich bevorzugt um Aufmerksamkeit, Konzentration, verbale Ausdrucksfähigkeit, Fähigkeit und Geschwindigkeit der Problemlösung, räumliches Vorstellungsvermögen, Planung und konzeptionelles Denken und Ausführung komplexer Tätigkeiten. Erfindungsgemäß bevorzugt eingesetzte Calendulaarten zur Herstellung der erfindungsgemäß verwendeten Extrakte sind *Calendula officinalis* und *Calendula arvensis*, besonders bevorzugt *Calendula officinalis,* wobei als bevorzugter Pflanzenteil die Blüten verwendet werden.

Calendula wird in die Familie der Asteraceae eingeordnet und umfasst in der Gattung etwa 12 Arten, welche vorwiegend im Mittelmeerraum verbreitet sind, wobei Calendula häufig aus dem Anbau stammt. Medizinisch verwendet werden die Arten *Calendula officinalis* und *Calendula arvensis.* Als Pflanzenteile werden verwendet die Blüten ("flos", im Fall von *Calendula officinalis* wird unterschieden zwischen "flos", vom Blütenstandsboden befreite Einzelblüten, bzw. "flos cum calice", Blütenkörbchen) und das Kraut ("herba", die zur Blütezeit gesammelten und getrockneten oberirdischen Teile) (HagerROM 2011, Springer-Verlag Heidelberg).

Die Hauptinhaltsstoffe von *Calendula officinalis* flos und flos cum calice sind oleanolsäurebasierte Triterpenglykoside (Saponine), Flavonoidglykoside, Phenolcarbonsäuren, Triterpenalkohole, sowie Sterole und einfache Cumarine. Als Nebeninhaltsstoffe sind Carotinoide, Sesquiterpene und Polysaccharide zu erwähnen. *Calendula officinalis* herba enthält ätherische Öle, Sterole, Carotinoide, Lycopin, Rubixanthin, Violaxanthin, Saponin, Phytosterin und den Bitterstoff Loliolid. Wichtige Inhaltsstoffe von *Calendula arvensis* flos sind Flavonoide, Carotinode und Lipide, wobei das Flavonoidspektrum dem von *Calendula officinalis* ähnelt. Die Hauptinhaltsstoffe von *Calendula arvensis* herba sind Oleanolsäureglykoside (Triterpensaponine), Sesquiterpenglykoside, Phenolcarbonsäuren und Aminosäuren (HagerROM 2011, Springer-Verlag Heidelberg).

Zubereitungen aus *Calendula officinalis* sind bisher überwiegend zur topischen Anwendung monographiert. Die Deutsche Kommission E und die europäische ESCOP Monographie benennen z.B. die äußerliche Anwendung bei Haut- und Schleimhautwunden, auch solchen mit schlechter Heilungstendenz, z.B. das Ulcus cruris. Aber auch innere, lokale Anwendungen bei entzündlichen Veränderungen der Mund- und Rachenschleimhaut sind beschrieben.
Die volkstümliche Anwendung von Calendula ist unter anderem aus Tunesien, Italien, Spanien, Kashmir, Russland und China bekannt. In der Volksmedizin wird Calendula dabei sowohl äußerlich als auch innerlich als entzündungshemmendes, fiebersenkendes, herzstärkendes, harntreibendes und schweißtreibendes Mittel eingesetzt, beispielsweise als Antirheumatikum, Choleretikum und Hypotensivum, sowie als Mittel gegen Angina, Husten, Krämpfe, Hautentzündungen und Menstruationsbeschwerden (vgl. HagerROM 2011, Springer-Verlag Heidelberg).

Wir haben nun überraschend gefunden, dass Extrakte aus Calendula bei innerer Anwendung kognitive und mentale Funktionen von Mäusen und Ratten deutlich verbessern, die durch eine experimentelle Schädigung oder durch den natürlichen Alterungsprozess beeinträchtigt waren.

So konnte an Mäusen gezeigt werden, dass eine durch Applikation von Scopolamin hervorgerufene Verschlechterung kognitiver und mentaler Funktionen durch Gabe von Calendulaextrakt aufgehoben werden kann. Die bei alten Mäusen auftretenden Einbußen mentaler Leistungen konnten ebenfalls durch orale Gabe des Extraktes komplett verhindert werden. An Ratten konnte in verschiedenen Versuchsmodellen, in denen die Gedächtnisfunktionen der Versuchstiere geschädigt wurden, ebenfalls eine protektive Wirkung des Calendulaextraktes gezeigt werden. Dabei wurde zum einen ein neuronales Schädigungsmodell verwendet, bei dem durch Gabe von Scopolamin spezifisch die cholinerge Neurotransmission verringert wird, und in einem weiteren, mehr globalen Schädigungsmodell, wurde die Energieversorgung des Gehirnes durch Applikation eines Mitochondriengiftes reduziert. Beide Modelle führen zu einer deutlichen Beeinflussung kognitiver und mentaler Funktionen, die in Verhaltensmodellen gemessen wurde.

Erfindungsgemäß werden Flüssigextrakte, Dickextrakte oder Trockenextrakte verwendet, wobei die Verwendung von Trockenextrakten bevorzugt ist. Gemäß dem Europäischen Arzneibuch haben Trockenextrakte im Allgemeinen einen Trockenrückstand von mindestens 95 Gew.-% und Flüssigextrakte sind flüssige Extraktzubereitungen in einem Lösungs- bzw. Extraktionsmittel. Dickextrakte sind halbfeste Zubereitungen, die durch (teilweises) Eindampfen des bei der Extraktion verwendeten Lösungs- bzw. Extraktionsmittels hergestellt werden.

Die erfindungsgemäß bevorzugt verwendeten Trockenextrakte sind in ihrer chemischen Zusammensetzung gekennzeichnet durch das Vorliegen hoher Gehalte an oleanolsäurebasierten Triterpenglykosiden, sowie an Flavonoidglykosiden. Des Weiteren enthalten die Extrakte qualitativ nahezu sämtliche vorstehend für *Calendula officinalis* flos bereits erwähnten Inhaltsstoffe, d.h. Phenolcarbonsäuren, Triterpenalkohole, Sterole und einfache Cumarine als weitere Hauptinhaltsstoffe neben oleanolsäurebasierten Triterpenglykosiden und Flavonoidglykosiden, sowie Carotinoide, Sesquiterpene und Polysaccharide als Nebeninhaltsstoffe.
Gemäß der vorliegenden Erfindung kann die orale Einnahme des Calendulaextraktes sowohl zur Prophylaxe bzw. Vorbeugung als auch zur Therapie bzw. Behandlung kognitiver und mentaler Störungen verwendet werden.

Zur Herstellung der im Rahmen der vorliegenden Erfindung verwendeten Extrakte aus Calendula wird das getrocknete und gemahlene Pflanzenmaterial mit einem organischen Lösungsmittel, Wasser, einem Gemisch aus mehreren organischen Lösungsmitteln oder einem Gemisch aus einem oder mehreren organischen Lösungsmitteln und Wasser bei einer Temperatur zwischen 10 °C und 100 °C unter Erhalt einer Extraktlösung extrahiert. Das ausextrahierte Pflanzenmaterial wird von der Extraktlösung, z. B. durch Filtration, abgetrennt und ggf. erneut mit einem Lösungsmittel oder Lösungsmittelgemisch entsprechend dem ersten Schritt extrahiert und ebenfalls von der Extraktlösung abgetrennt. Die so gewonnenen Extraktlösungen werden vereinigt, eingedampft und getrocknet.

Bevorzugte organische Lösungsmittel für die Extraktion sind Alkohole oder Ketone, vorzugsweise Ethanol oder Aceton und deren Gemische mit Wasser. Besonders bevorzugte Lösungsmittel für die Extraktion sind Gemische aus Ethanol und Wasser im Gewichtsverhältnis von 20/80 bis 80/20 (20 Gew.-% bis 80 Gew.-% Ethanol), vorzugsweise 50/50 bis 70/30 (50 Gew.-% bis 70 Gew.-% Ethanol), besonders bevorzugt 60/40 (60 Gew.-% Ethanol), sowie Wasser. Bei der Verwendung eines Gemisches aus Wasser und Ethanol als das Lösungsmittel für die Extraktion wird eine wässrig-ethanolische Extraktlösung erhalten. Dieser Extrakt als auch der daraus durch Trocknung gewonnene Extrakt wird als wässrig-ethanolischer Extrakt bezeichnet. Bei der Verwendung von Wasser als das Lösungsmittel für die Extraktion wird eine wässrige Extraktlösung erhalten. Dieser Extrakt als auch der daraus durch Trocknung gewonnene Extrakt wird als wässriger Extrakt bezeichnet. Als Extraktionsverfahren kommen z. B. Mazeration oder Perkolation in Frage (vgl. Europäisches Arzneibuch, Ausgabe 6.0). Die Trocknung kann durch an sich bekannte Verfahren wie Gefriertrocknung oder Trocknung im Vakuum bei Raumtemperatur oder erhöhter Temperatur erfolgen. Zur Anreicherung von ausgewählten Inhaltsstoffen können weitere Konzentrierungsschritte durchgeführt werden wie flüssig-flüssig-Verteilung mit z. B. 1-Butanol/Wasser oder Ethylacetat/Wasser, Adsorption-Desorption an Ionenaustauschern oder anderen Harzen, wie Sephadex LH20 oder Diaion HP20, oder chromatographische Abtrennungen über RP18, Kieselgel, etc..

In einer bevorzugten Ausführungsform wird ein Gewichtsteil gemahlene Blüten der gewünschten Calendula-Art mit fünf bis zehn Gewichtsteilen eines Gemisches aus Ethanol und Wasser (50 Gew.-% bis 70 Gew.-% Ethanol) ½ Std. bis 3 Std. bei 50°C bis 60 °C unter Erhalt einer Extraktlösung gerührt. Das ausextrahierte Pflanzenmaterial wird von der Extraktlösung durch Filtration abgetrennt und erneut mit fünf bis zehn Gewichtsteilen eines Gemisches aus Ethanol und Wasser (50 Gew.-% bis 70 Gew.-% Ethanol) ½ Std. bis 3 Std. bei 50 °C bis 60 °C gerührt und abfiltriert. Die vereinigten Filtrate aus den beiden Extraktionsschritten werden im Vakuum bei 40 °C bis 60 °C vom Ethanol befreit und gefriergetrocknet und/oder im Vakuum bei 40 °C bis 60 °C im Trockenschrank getrocknet. Dabei werden die erfindungsgemäß bevorzugt verwendeten Trockenextrakte mit einem Wassergehalt von weniger als 5 Gew.-% erhalten.

Vorzugsweise wird der Extrakt in Form eines Mittels, insbesondere eines Arzneimittels oder Lebensmittels, das einen Extrakt aus Calendula umfasst, verwendet. Die Extrakte können in Form von Tropfen, Pulvern, Granulaten, Tabletten, Dragees oder Kapseln vorzugsweise oral verabreicht werden. Möglich ist aber auch eine parenterale Anwendung in Form einer Injektionslösung oder eine topische Anwendung in Form von Cremes, Salben, Suppositorien, Pflastern oder ähnlichen Zubereitungen.

Zur Herstellung von Tabletten wird der Extrakt mit geeigneten pharmazeutisch bzw. lebensmittelrechtlich akzeptablen Hilfsstoffen, z. B. Laktose, Cellulose, Siliciumdioxid, Croscarmellose und Magnesiumstearat, gemischt und zu Tabletten gepresst, die gegebenenfalls mit einem geeigneten Überzug, z. B. aus Hydroxymethylpropylcellulose, Polyethylenglykol, Farbstoffen (z. B. Titandioxid, Eisenoxid) und Talkum, versehen werden.

Die Extrakte können auch, ggf. unter Zusatz von pharmazeutisch bzw. lebensmittelrechtlich akzeptablen Hilfsstoffen wie Stabilisatoren, Füllmittel etc., in Kapseln abgefüllt werden.

Die Dosierung erfolgt dabei so, dass pro Tag 5 bis 2000 mg, bevorzugt 10 bis 1000 mg, besonders bevorzugt 60 bis 600 mg Extrakt zugeführt werden.

Die Extrakte sowie die daraus hergestellten Mittel, die einen Extrakt aus Calendula umfassen, können sowohl als Arzneimittel als auch als Lebensmittel verwendet werden. Unter Lebensmittel sind hierbei insbesondere diätetische Lebensmittel, Nahrungsergänzungsmittel sowie "medical food" und "dietary supplements" zu verstehen.

Die pharmakologischen Wirkungen auf kognitive und mentale Funktionen wurden in verschiedenen Tiermodellen untersucht.

### 1. Untersuchungen im T-Maze:

Mäuse und Ratten zeigen im sogenannten T-Maze Model eine natürliche Tendenz zum alternierenden Wechsel der beiden Labyrinth-Arme. Dieses angeborene Verhalten wird durch das Kurzzeitgedächtnis gesteuert. Abhängig davon, was gerade vorher getan wurde, werden auch räumliche Gedächtnisfunktionen beeinflusst. Das ständige Wechseln zwischen den zwei möglichen Alternativen wird vermutlich durch den Trieb der Tiere zur Erkundung der Umgebung und der Suche nach verschiedenen Resourcen, wie Nahrung, Wasser oder Artgenossen bestimmt. Typische, in der Literatur genannte, Wechselraten liegen bei ca. 75% (R. M. J. Deacon, J. N. P. Rawlins, "T-Maze alternation in the rodent", Nat. Prot. 1, 7 - 12; 2006).

Um eine Verbesserung kognitiver und mentaler Funktionen messen zu können, wurde Mäusen das Anticholinergikum Scopolamin appliziert, was zu Gedächtnisstörungen führt.

Die zentrale cholinerge Neurotransmission spielt eine fundamentale Rolle in der Physiologie und Pathophysiologie kognitiver und mentaler Funktionen. So führt beispielsweise die Aktivierung cholinerger Systeme im Gehirn in tierpharmakologischen Experimenten aber auch in der klinischen Anwendung zu einer Verbesserung der Gedächtnisleistungen (N. Narimatsu, N. Harada, H. Kurihara, N. Nakagata, K. Sobue, K. Okajima, "Donepezil improves cognitive function in mice by increasing the production of insulin-like growth factor-I in the hippocampus", J. Pharmacol. Exp. Ther. 330, 2 - 12; 2009). Eine Schädigung cholinerger Aktivitäten durch Applikation eines Anticholinergikums wie Scopolamin ist dagegen ein etabliertes pharmakologisches Modell zur Induktion von kognitiven und mentalen Fehlfunktionen in Tieren und ist somit geeignet, Substanzwirkungen zur Verbesserung kognitiver und mentaler Funktionen zu detektieren (N. M. W. J. De Bruin, J. Prickaerts, J. H. M. Lange, S. Akkerman, E. Andriambeloson, M. de Haan, J. Wijnen, M. van Drimmelen, E. Hissink, L. Heijink, C. G. Kruse, "SLV330, a cannabinoid CB1 receptor antagonist, ameliorates deficits in the T-maze, object recognition and social recognition tasks in rodents", Neurobiol. Learn. Mem. 93, 522 - 531, Epub 2010 Feb 2; 2010).

Der erfindungsgemäß verwendete Calendula-Extrakt gemäß Beispiel 1 wurde in den Dosierungen 150 und 300 mg/kg 60 Minuten vor dem Test oral per Schlundsonde verabreicht. Scopolamin (1 mg/kg) wurde 20 Minuten vor dem Test intraperitoneal (ip) verabreicht. Donepezil wurde als positive Referenzsubstanz zur Antagonisierung der Scopolaminwirkung in einer Dosierung von 0,3 mg/kg ebenfalls intraperitoneal 20 Minuten vor Testdurchführung verabreicht.

Scopolamin verursacht eine deutliche Reduktion des Wechselverhaltens. Donepezil hebt die Wirkung von Scopolamin weitgehend auf und zeigt so die Validität der Untersuchungsergebnisse. Der Calendula-Extrakt gemäß Beispiel 1 zeigt in beiden Dosierungen (150 mg/kg und 300 mg/kg) eine dem Donepezil vergleichbare und ebenfalls statistisch signifikante Hemmung der Scopolamineffekte (Irrtumswahrscheinlichkeit p < 0,01).

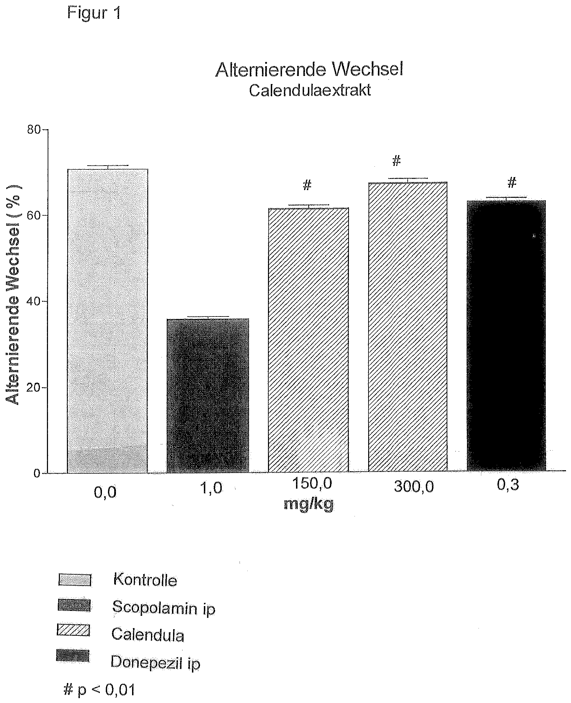

Das spontane Wechselverhalten im T-Maze Modell (Maus) ist in Figur 1 gezeigt.

### 2. Untersuchungen im Morris Water Maze an der Ratte:

Das Morris Water Maze Modell wurde von Richard Morris zur Messung des räumlichen Lernens von Versuchstieren entwickelt und 1984 publiziert (R. Morris, "Developments of a water-maze procedure for studying spatial learning in the rat", J. Neurosci. Methods 11, 47 - 60; 1984). Dabei müssen Ratten in einem Swimmingpool (Durchmesser 180 cm), der mit 22 °C temperierten Wasser gefüllt ist, viermal täglich von verschiedenen Startpositionen aus innerhalb von maximal 60 Sekunden eine Plattform finden, die sich 1,5 cm unterhalb des Wasserspiegels befindet. Damit ist sie für die Tiere nicht sichtbar, allerdings helfen fixe Markierungsmarken im Raum bei der räumlichen Orientierung. Sollte die Ratte innerhalb der 60 Sekunden die Plattform nicht finden, wird sie vom Experimentator zur Plattform geführt und für 30 Sekunden auf dieser belassen, bevor der nächste Durchgang beginnt. Nach drei Tagen Training mit täglich vier Übungszyklen finden ca. 80% bis 100% der Tiere die Plattform.

Um eine der menschlichen Demenz vergleichbare Einschränkung der Lernleistung hervorzurufen, wurde ein globales Schädigungsmodell verwendet. Dazu wurde den Ratten zu Versuchsbeginn in Vollnarkose eine osmotische Pumpe subkutan implantiert, die über einen Zeitraum von 28 Tagen eine gleichbleibende Menge von Natriumazid abgibt, was zu einer fortschreitenden Einschränkung der mitochondrialen Funktionen und damit einhergehend zu einer Reduktion der Energieversorgung des Gehirns führt und somit als Demenzmodel geeignet ist (T. Szabados, C. Dul, K. Majtenyi, M. Hargitai, Z. Penzes, R. Urbanics, "A chronic Alzheimer's model evoked by mitochondrial poison sodium azide for pharmacological investigations", Behav. Brain Res. 154, 31 - 40; 2004). Die Lernfähigkeit dieser Tiere wurde dann im Morris Watermaze Modell an den Versuchstagen 13, 14 und 15 getestet und die Gedächtnisfähigkeit an Versuchstag 20 überprüft.

| | |
|---|---|
| 1. Gruppe Kontrolle | (tägliche Behandlung mit Agarosegel als Vehikel und osmotische Pumpe mit physiologischer Kochsalzlösung gefüllt). |
| 2. Gruppe Natriumazid | (tägliche Behandlung mit Agarosegel als Vehikel und osmotische Pumpe mit Natriumazid gefüllt). |
| 3-5. Gruppe Calendula | (tägliche Behandlung mit 50 (Gruppe 3), 100 (Gruppe 4) bzw. 200 (Gruppe 5) mg/kg/Tag Calendula-Extrakt gemäß Beispiel 1 und osmotische Pumpe mit Natriumazid gefüllt). |

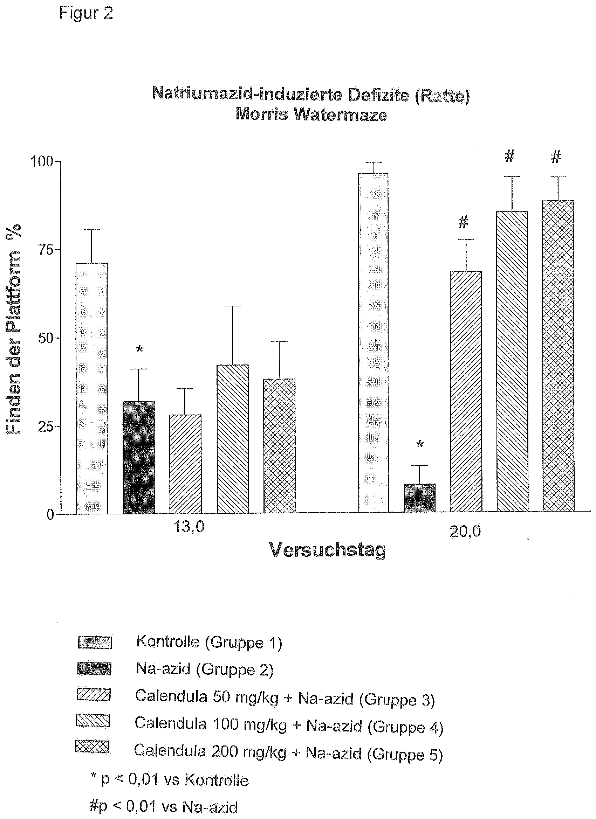

Der erfindungsgemäß verwendete Calendula-Extrakt wurde in den Dosierungen 50, 100 und 200 mg/kg einmal täglich oral per Schlundsonde verabreicht. Am Tag 13 nach Beginn der Natriumazid- und Extraktbehandlung wurden die Ratten erstmalig im Morris Water Maze trainiert, die verborgene Plattform zu finden (Ausgangswert). An den Versuchstagen 14 und 15 wurden die Tiere ebenfalls trainiert (in der Grafik aus Übersichtsgründen nicht dargestellt), und am 20. Versuchstag wurde der Lernerfolg kontrolliert. Wie der Grafik zu entnehmen ist, finden die Kontrolltiere bereits am ersten Trainingstag deutlich häufiger die Plattform als die mit Natriumazid behandelten Gruppen. Fünf Tage nach Beendigung der dreitägigen Trainingsphase finden 95% der Kontrolltiere (Gruppe 1), 8% der Natriumazid-behandelten Tiere (Gruppe 2), und 68% (50 mg/kg/Tag Calendula-Extrakt, Gruppe 3), 85% (100 mg/kg/Tag Calendula-Extrakt, Gruppe 4) und 88% (200 mg/kg/Tag Calendula-Extrakt, Gruppe 5) der mit Natriumazid und mit Calendulaextrakt gemäß Beispiel 1 behandelten Tiere die Plattform innerhalb von 60 Sekunden. Der Effekt war in allen drei untersuchten Dosierungen statistisch signifikant (Student-t-Verteilung, Irrtumswahrscheinlichkeit p < 0,01).
Die Ergebnisse der Untersuchungen im Morris Water Maze (Natriumazid-induzierte Schädigung) an der Ratte sind in Figur 2 gezeigt.

### Beispiel 1: Extrakt aus Calendula officinalis

500 g fein gemahlene Blüten (flos cum calice) von *Calendula officinalis* werden zwei mal mit je 3500 g 60 Gew.-% Ethanol jeweils 1 Std. bei 60 °C gerührt, anschließend wird die Suspension über ein Seitz Supra 1500 Filter abgesaugt und die vereinigten Filtrate werden im Vakuum bei 60 °C vom Lösemittel befreit. Der verbliebene Rückstand wird über Nacht im Vakuumtrockenschrank bei 50°C nachgetrocknet: 205,8 g (41,2%) Trockenextrakt.

### Beispiel 2: Tabletten

Ein Trockenextrakt aus *Calendula officinalis* (Extrakt gemäß Beispiel 1) wird mit Hilfsstoffen gemischt und zu Tabletten verpresst (Tablettenkern = Bestandteil 1 - 6). Die Tabletten werden mit einem Überzug aus Hydroxypropylmethylcellulose versehen (Bestandteil 7 -10).

| | *Bestandteil* | *mg*/*Tablette* |
|---|---|---|
| 1 | Trockenextrakt aus *Calendula officinalis* gemäß Beispiel 1 | 100,0 |
| 2 | Mikrokristalline Cellulose | 117,0 |
| 3 | Laktose-Monohydrat | 58,0 |
| 4 | Croscarmellose | 15,0 |
| 5 | Hochdisperses Siliciumdioxid | 3,0 |
| 6 | Magnesiumstearat | 6,0 |
| 7 | Hydroxypropylmethylcellulose | 15,0 |
| 8 | Polyethylenglycol | 3,0 |
| 9 | Talkum | 1,0 |
| 10 | Titandioxid | 2,0 |

## Patentansprüche

1. Extrakt aus Calendula zur Verwendung in der Behandlung und Vorbeugung von Störungen und Beeinträchtigungen kognitiver und mentaler Funktionen.

2. Extrakt zur Verwendung nach Anspruch 1, wobei es sich bei den Störungen und Beeinträchtigungen kognitiver und mentaler Funktionen um demenzielle Syndrome sowie deren Frühformen und Vorstufen handelt.

3. Extrakt zur Verwendung nach Anspruch 2, wobei die demenziellen Syndrome ausgewählt sind aus leichten bis schweren Formen der Alzheimer-Demenz, der vaskulären Demenz und deren Mischformen.

4. Extrakt zur Verwendung nach Anspruch 2, wobei es sich bei den Frühformen und Vorstufen der demenziellen Syndrome um leichte kognitive Beeinträchtigungen (MCI), kognitive Beeinträchtigungen, die noch keine Demenz darstellen (CIND), sowie objektive oder subjektiv empfundene Gedächtnisbeeinträchtigungen und Beeinträchtigungen anderer kognitiver Leistungskomponenten, Apathie, Antriebsmangel und andere demenzielle Symptome handelt.

5. Extrakt zur Verwendung nach Anspruch 4, wobei die anderen subjektiv und/oder objektiv beeinträchtigten kognitiven Leistungskomponenten ausgewählt sind aus Aufmerksamkeit, Konzentration, verbaler Ausdrucksfähigkeit, Fähigkeit und Geschwindigkeit der Problemlösung, räumlichem Vorstellungsvermögen, Planung und konzeptionellem Denken und Ausführung komplexer Tätigkeiten.

6. Extrakt zur Verwendung nach einem der Ansprüche 1 bis 5, wobei als Calendulaart zur Herstellung des Extraktes Calendula officinalis oder Calendula arvensis eingesetzt wird.

7. Extrakt zur Verwendung nach einem der Ansprüche 1 bis 6, wobei zur Herstellung des Extraktes die Blüten von Calendula eingesetzt werden.

8. Extrakt zur Verwendung nach Anspruch 7, wobei zur Herstellung des Extraktes die Blüten von Calendula officinalis eingesetzt werden.

9. Extrakt zur Verwendung nach einem der Ansprüche 1 bis 8, wobei zur Herstellung des Extraktes als Lösungsmittel für die Extraktion Ethanol und Wasser im Gewichtsverhältnis von 20/80 bis 80/20 eingesetzt werden.

10. Extrakt zur Verwendung nach Anspruch 9, wobei zur Herstellung des Extraktes als Lösungsmittel für die Extraktion Ethanol und Wasser im Gewichtsverhältnis von 50/50 bis 70/30 eingesetzt werden.

11. Extrakt zur Verwendung nach Anspruch 10, wobei zur Herstellung des Extraktes als Lösungsmittel für die Extraktion Ethanol und Wasser im Gewichtsverhältnis von 60/40 eingesetzt werden.

12. Extrakt zur Verwendung nach einem der Ansprüche 1 bis 8, wobei zur Herstellung des Extraktes als Lösungsmittel für die Extraktion Wasser eingesetzt wird.

13. Extrakt zur Verwendung nach einem der Ansprüche 1 bis 12, wobei der Extrakt ein Trockenextrakt ist.

14. Extrakt zur Verwendung nach einem der Ansprüche 1 bis 13, wobei die Verabreichung in Form von Tropfen, Pulvern, Granulaten, Tabletten, Dragees oder Kapseln erfolgt.

15. Extrakt zur Verwendung nach einem der Ansprüche 1 bis 14, wobei die Verabreichung des Extraktes oral erfolgt.

16. Extrakt zur Verwendung nach einem der Ansprüche 1 bis 15, wobei die Verabreichung des Extraktes in der Form eines Mittels, das einen Extrakt aus Calendula umfasst, erfolgt.

17. Extrakt zur Verwendung nach Anspruch 16, wobei das Mittel ein Arzneimittel oder ein Lebensmittel ist.

18. Extrakt zur Verwendung nach einem der Ansprüche 1 bis 17, wobei die Verabreichung des Extraktes in einer Dosierung von 5 bis 2000 mg Extrakt pro Tag erfolgt.

## Claims

1. Extract of Calendula for use in the treatment and prevention of disorders and impairments of cognitive and mental functions.

2. Extract for use according to claim 1, wherein the disorders and impairments of cognitive and mental functions are dementia syndromes as well as their early forms and preliminary stages.

3. Extract for use according to claim 2, where the dementia syndromes are selected from mild to severe forms of Alzheimer's disease, vascular dementia and mixed forms thereof.

4. Extract for use according to claim 2, where the early forms and preliminary stages of the dementia syndromes are mild cognitive impairments (MCI), cognitive impairments which are not yet dementia (CIND) as well as objectively or subjectively perceived memory impairments and impairments of other cognitive performance related components, apathy, lack of motivation and other dementia syndromes.

5. Extract for use according to claim 4, where the other subjectively and/or objectively impaired cognitive performance related components are selected from attention, concentration, verbal expressions, skill and speed of solving problems, spatial imagination, planning and conceptual thinking and performing of complex tasks.

6. Extract for use according to any one of claims 1 to 5, wherein the species of Calendula used for the manufacture of the extract is Calendula officinalis or Calendula arvensis.

7. Extract for use according to any one of claims 1 to 6, wherein the flowers of Calendula are used for the manufacture of the extract.

8. Extract for use according to claim 7, wherein the flowers of Calendula officinalis are used for the manufacture of the extract.

9. Extract for use according to any one of claims 1 to 8, wherein ethanol and water in a weight ratio of 20/80 to 80/20 is used as solvent for the extraction in the manufacture of the extract.

10. Extract for use according to claim 9, wherein ethanol and water in a weight ratio of 50/50 to 70/30 are used as solvent for the extraction in the manufacture of the extract.

11. Extract for use according to claim 10, wherein ethanol and water in a weight ratio of 60/40 are used as solvent for the extraction in the manufacture of the extract.

12. Extract for use according to any one of claims 1 to 8, wherein water is used as the solvent for the extraction in the manufacture of the extract.

13. Extract for use according to any one of claims 1 to 12, wherein the extract is a dry extract.

14. Extract for use according to any one of claims 1 to 13, wherein the administration is in the form of drops, powders, granules, tablets, dragées or capsules.

15. Extract for use according to any one of claims 1 to 14, wherein the administration of the extract is oral.

16. Extract for use according to any one of claims 1 to 15, wherein the administration of the extract is in the form of a composition which contains a Calendula extract.

17. Extract for use according to claim 16, wherein the composition is a drug or a food product.

18. Extract for use according to any one of claims 1 to 17, wherein the administration of the extract is at a dose of 5 to 2000 mg extract per day.

## Revendications

1. Un extrait de calendula pour l'utilisation dans le traitement et la prévention de troubles et d'entraves de fonctions cognitives et mentales.

2. L'extrait pour l'utilisation selon la revendication 1, les troubles et les entraves de fonctions cognitives et mentales étant des syndromes de démence ainsi que de leurs formes précoces et leurs stades préliminaires.

3. L'extrait pour l'utilisation selon la revendication 2, où les syndromes de démence sont choisis parmi les formes légères à sévères de la démence d'Alzheimer, de la démence vasculaire et des formes mixtes de ceux-ci.

4. L'extrait pour l'utilisation selon la revendication 2, où les formes précoces et les stades préliminaires des syndromes de démence sont des déficiences cognitives légères (MCI), des déficiences cognitives, qui ne constituent pas encore la démence (CIND), ainsi que la perception objective ou subjective des entraves de la mémoire et des entraves d'autres composants de puissance cognitive, l'apathie, le manque de motivation et d'autres symptômes de démence.

5. L'extrait pour l'utilisation selon la revendication 4, où les autres composants de puissance cognitive, qui sont perçus subjectivement ou objectivement comme entravés, sont choisis parmi l'attention, la concentration, les compétences en communication verbale, la capacité et la vitesse de la résolution de problèmes, l'imagination spatiale, la planification et la pensée conceptuelle et l'exécution d'activités complexes.

6. L'extrait pour l'utilisation selon l'une quelconque des revendications 1 à 5, où calendula officinalis ou calendula arvensis est utilisé pour la préparation de l'extrait.

7. L'extrait pour l'utilisation selon l'une quelconque des revendications 1 à 6, où les fleurs de calendula sont utilisées pour la préparation de d'extrait.

8. L'extrait pour l'utilisation selon la revendication 7, où les fleurs de calendula officinalis sont utilisées pour la préparation de l'extrait.

9. L'extrait pour l'utilisation selon l'une quelconque des revendications 1 à 8, où, pour la préparation de l'extrait, de l'éthanol et de l'eau sont utilisés dans un rapport pondéral de 20/80 à 80/20 en tant que solvant pour l'extraction.

10. L'extrait pour l'utilisation selon la revendication 9, où, pour la préparation de l'extrait, de l'éthanol et de l'eau sont utilisés dans un rapport pondéral de 50/50 à 70/30 en tant que solvant pour l'extraction.

11. L'extrait pour l'utilisation selon la revendication 10, où, pour la préparation de l'extrait, de l'éthanol et de l'eau sont utilisés dans un rapport pondéral de 60/40 en tant que solvant pour l'extraction.

12. L'extrait pour l'utilisation selon l'une quelconque des revendications 1 à 8, où, pour la préparation de l'extrait, de l'eau en tant que solvant pour l'extraction est utilisée.

13. L'extrait pour l'utilisation selon l'une quelconque des revendications 1 à 12, où l'extrait est un extrait sec.

14. L'extrait pour l'utilisation selon l'une quelconque des revendications 1 à 13, où l'administration est sous forme de gouttes, de poudres, de granulés, de comprimés, de dragées ou de capsules.

15. L'extrait pour l'utilisation selon l'une quelconque des revendications 1 à 14, où l'administration est par voie orale.

16. L'extrait pour l'utilisation selon l'une quelconque des revendications 1 à 15, où l'administration de l'extrait se fait sous forme d'un agent, qui comprend un extrait de calendula.

17. L'extrait pour l'utilisation selon la revendication 16, où l'agent est un médicament ou un aliment.

18. L'extrait pour l'utilisation selon l'une quelconque des revendications 1 à 17, où l'extrait est administré à une posologie de 5 à 2000 mg d'extrait par jour.
